# EUROPEAN PATENT APPLICATION

(11) **EP 4 365 278 A1**
(43) Date of publication of application: **08.05.2024**
(21) Application number: 22832635.1
(22) Date of filing: 17.05.2022
(51) Int. Cl.: C12M 1/34, C12Q 1/6869, C12Q 1/689, A01K 29/00

(54) **DISEASE INCIDENCE PREDICTION SYSTEM, INSURANCE PREMIUM CALCULATION SYSTEM, METHOD FOR PREDICTING DISEASE INCIDENCE, AND METHOD FOR CALCULATING INSURANCE PREMIUM**

(30) Priority: 30.06.2021 JP 2021109714
(71) Applicant: Anicom Holdings, Inc., Shinjuku-ku Tokyo 160-0023 (JP)
(72) Inventor: MATSUMOTO, Kengo, Tokyo 160-0023 (JP); SUMI, Hiroshi, Tokyo 160-0023 (JP); HORIE, Ryo, Tokyo 160-0023 (JP)
(74) Representative: Gill Jennings & Every LLP
(86) International application number: PCT/JP2022/020564
(87) International publication number: WO 2023/276476

(57) **Abstract**

An objective of the present invention is to provide a system for predicting disease contraction and a method for predicting disease contraction for predicting whether there is a possibility that an animal will contract a specific disease.

The system for predicting disease contraction is provided with: a reception means for receiving information on the presence/absence of bacteria that belong to Fusobacteriaceae and/or Veillonellaceae in a sample collected from an animal; and an assessment means for performing predictive assessment regarding the possibility that the animal will contract keratoconjunctivitis sicca from the information, input to the reception means, on the presence/absence of bacteria that belong to Fusobacteriaceae and/or Veillonellaceae.

## Description

### TECHNICAL FIELD

The present invention relates to a system for predicting disease contraction, an insurance fee calculation system, a method for predicting disease contraction and an insurance fee calculation method. Specifically, the invention relates to a system for predicting disease contraction and a method for predicting disease contraction for providing predictive information on whether an animal will contract a specific disease from data on whether the animal carries bacteria that belong to a specific bacteria family, and an insurance fee calculation system and an insurance fee calculation method for calculating or correcting an insurance fee of an animal from data on whether the animal carries bacteria that belong to a specific bacteria family.

### BACKGROUND ART

Pets such as dogs, cats or rabbits, and livestock such as cattle and pigs, are irreplaceable to humans. In recent years, while the average life of animals raised by humans has been greatly extended, animals more often contract any disease during their lifetime, and there is a problem of an increase in the medical expenses borne by keepers.

In order to maintain the health of an animal, physical condition management through daily meals, exercise and the like and quick action in response to physical disorder is important. An animal, however, is unable to describe the disorder of the body in its own words, and thus, the keeper is aware of the contraction of a disease by the animal for the first time when a sign that can be observed externally has appeared with progressing symptom presentation. If it is possible to grasp the possibility that an animal will contract a disease, it would be possible to take action to avoid such a disease.

Thus, there is required a means for learning on, by means of a simple method, the possibility that an animal may contract a disease in the future.

Keratoconjunctivitis sicca (KCS) is an ophthalmic disease that causes a disorder of cornea or conjunctive by a decrease in tear volume. Dry eye causes conjunctival hyperemia or corneal inflammation, scar or pigmentation, or the like. Allergic dermatitis refers to dermatitis caused by an excessive reaction of an immune system in the animal body by an allergen as a causative agent of allergic symptoms. Allergens include fleas, house dust, pollen and foods. Inflammatory bowel disease (IBD) is a chronic digestive disease with an unknown cause characterized by the infiltration of inflammatory cells into the intestinal mucosa. IBD is classified into "lymphocyte plasma cell enteritis", "lymphocyte plasma cell colitis", "eosinophilic gastroenteritis", "granulomatous enterocolitis", "histiocytic ulcerative enteritis" and the like, in accordance with the type and location of infiltrating inflammatory cells.

Patent Document 1 discloses an intestinal microflora-regulating composition or an intestinal microflora-improving composition that has an effect of effectively regulating or improving an intestinal microflora by growing bacteria of the phylum of Bacteroidetes and reducing bacteria of the phylum of Firmicutes in the intestinal microflora, but does not disclose a method for predicting whether there is a possibility that an animal will contract a specific disease from data on whether the animal carries bacteria that belong to a specific bacteria family.

### RELATED ART DOCUMENTS

### PATENT DOCUMENTS

[Patent Document 1] WO 2017/094892

### SUMMARY OF THE INVENTION

### PROBLEMS TO BE SOLVED BY THE INVENTION

The invention aims to provide a system for predicting disease contraction and a method for predicting disease contraction for predicting the possibility that an animal will contract a specific disease by way of a simple method.

### MEANS FOR SOLVING THE PROBLEMS

The present inventors have analyzed and investigated data on bacteria carried by animals with pet insurance, in particular data on intestinal microflora, and huge data on whether the animal has contracted certain diseases and whether an insurance claim has been made for the animal. As a result, the inventors have found that it is possible to predict, from the data on bacteria carried by the animal, the possibility that the animal will contract a specific disease, and have completed the present invention.

The invention includes the following [1] to [10]:
[1] A system for predicting disease contraction including: a reception means for receiving information on the presence/absence of bacteria belonging to Fusobacteriaceae and/or Veillonellaceae in a sample collected from an animal; and an assessment means for performing predictive assessment regarding the possibility of the animal contracting keratoconjunctivitis sicca from the information, input to the reception means, on the presence/absence of bacteria belonging to Fusobacteriaceae and/or Veillonellaceae.
[2] A system for predicting disease contraction including: a reception means for receiving information on the presence/absence of bacteria belonging to Fusobacteriaceae and/or Veillonellaceae in a sample collected from an animal; and an assessment means for performing a predictive assessment regarding the possibility of the animal contracting allergic dermatitis from the information, input to the reception means, on the presence/absence of bacteria belonging to Fusobacteriaceae and/or Veillonellaceae.
[3] A system for predicting disease contraction including: a reception means for receiving information on the presence/absence of bacteria belonging to Fusobacteriaceae and/or Veillonellaceae in a sample collected from an animal; and an assessment means for performing predictive assessment regarding the possibility of the animal contracting inflammatory bowel disease from the information, input to the reception means, on the presence/absence of bacteria belonging to Fusobacteriaceae and/or Veillonellaceae.
[4] The system for predicting disease contraction according to any one of [1] to [3], wherein the sample is feces.
[5] An insurance fee calculation system including: a reception means for receiving information on the presence/absence of bacteria belonging to Fusobacteriaceae and/or Veillonellaceae in a sample collected from an animal; and an insurance fee calculation means for calculating or correcting an insurance fee using the information, input to the reception means, on the presence/absence of bacteria belonging to Fusobacteriaceae and/or Veillonellaceae.
[6] A method for predicting disease contraction including performing predictive assessment regarding the possibility of an animal contracting keratoconjunctivitis sicca from information on the presence/absence of bacteria belonging to Fusobacteriaceae and/or Veillonellaceae in a sample collected from the animal.
[7] A method for predicting disease contraction including performing predictive assessment regarding the possibility of an animal contracting allergic dermatitis from information on the presence/absence of bacteria belonging to Fusobacteriaceae and/or Veillonellaceae in a sample collected from the animal.
[8] A method for predicting disease contraction including performing predictive assessment regarding the possibility of an animal contracting inflammatory bowel disease from information on the presence/absence of bacteria belonging to Fusobacteriaceae and/or Veillonellaceae in a sample collected from the animal.
[9] The method for predicting disease contraction according to any one of [6] to [8], wherein the sample is feces.
[10] An insurance fee calculation method including calculating or correcting an insurance fee using information on the presence/absence of bacteria belonging to Fusobacteriaceae and/or Veillonellaceae in a sample collected from an animal.

### EFFECTS OF THE INVENTION

The invention makes it possible to provide a system for predicting disease contraction and a method for predicting disease contraction for predicting whether there is a possibility that an animal will contract a specific disease. The presence/absence of bacteria that belong to Fusobacteriaceae and/or Veillonellaceae in a sample collected from an animal is related to the possibility of a future insurance claim of the animal. It is thus possible to provide a system for predicting disease contraction and a method for predicting disease contraction capable of calculating or correcting an insurance fee of an animal using information on the presence/absence of bacteria that belong to Fusobacteriaceae and/or Veillonellaceae.

### BRIEF DESCRIPTION OF THE DRAWINGS

FIG. 1 is a schematic diagram of an embodiment of a system for predicting disease contraction according to the invention.
FIG. 2 is a flowchart of an example of the flow of a method for predicting disease contraction carried out by the system for predicting disease contraction according to the invention.
FIG. 3 is a schematic diagram of an embodiment of an insurance fee calculation system according to the invention.
FIG. 4 is a flowchart of an example of the flow of an insurance fee calculation method according to the invention.
FIG. 5 is a graph of the results of Examples.
FIG. 6 is a graph of the results of Examples.
FIG. 7 is a graph of the results of Examples;
FIG. 8 is a graph of the results of Examples.
FIG. 9 is a graph of the results of Examples.

### MODE FOR CARRYING OUT THE INVENTION

### <System for predicting disease contraction>

The system for predicting disease contraction according to the invention includes: a reception means for receiving information on the presence/absence of bacteria that belong to Fusobacteriaceae and/or Veillonellaceae in a sample collected from an animal; and an assessment means for performing predictive assessment regarding the possibility that the animal will contract keratoconjunctivitis sicca, allergic dermatitis or inflammatory bowel disease (hereinafter collectively referred to as a "specific disease") from the information, input to the reception means, on the presence/absence of bacteria that belong to Fusobacteriaceae and/or Veillonellaceae.

### [Reception means]

The reception means according to the invention is a means for receiving input of information on the presence/absence of bacteria that belong to Fusobacteriaceae and/or Veillonellaceae in a sample collected from an animal of which possibility of contracting a specific disease is to be predicted. The animal may be a dog, a cat, a bird, a rabbit, a ferret or the like. The age of a target animal is not limited. A method for receiving data on the presence/absence or hit rate of bacteria may be any method, including input or transmission of data to a terminal. A sample collected from an animal may be salve or feces, and feces is preferable. It is possible to confirm the presence/absence of bacteria that belong to Fusobacteriaceae and/or Veillonellaceae in an intestinal microflora of an animal by analyzing feces of the animal.

The presence/absence of bacteria that belong to Fusobacteriaceae and/or Veillonellaceae in a sample collected from an animal may be confirmed by applying, to a sample collected from an animal, such as a saliva sample or a feces sample, a known metagenomic analysis method, for example, the amplicon sequence method using a sequencer such as NGS or a bacterial flora analysis method. For example, there may be used a method for identifying an organism contained in a sample by analyzing the base sequence information of on DNA or RNA of any organism contained in the sample using a next-generation sequencer. Preferably, there is a method where all or part of 16SrRNA genes contained in a sample are amplified as required to perform sequencing and the sequence acquired is analyzed with software to obtain composition data of bacteria in the sample. By processing the composition data in a sample with software or referencing gene databases such as Genbank, Greengenes, and SILVA database, the assignment of bacteria species of bacteria contained in a sample is determined to measure the presence/absence and hit rate of bacteria that belong to a specific family.

An example of the amplicon analysis (bacterial flora analysis) of 16SrRNA genes using an NGS (next-generation sequencer) is specifically described below. First, DNA is extracted from a sample using a DNA extraction reagent and 16SrRNA genes are amplified with PCR from the extracted DNA. NGS is used to exhaustively determine a base sequence of the amplified DNA segments. After low quality reads and chimeric sequences are removed, the sequences are clustered and an OTU (Operational Taxonomic Unit) analysis is made. An OTU is a taxonomic unit for operating purposes used to treat sequences having more than a certain level of similarity (for example a homology of 96 to 97 percent or more) to each other as if they were a single bacteria species. It is thus considered that the number of OTUs represents the number of bacteria species constituting a bacterial flora and the number of reads that belong to the same OTU represents a relative existence amount of that species. Further, it is possible to select a typical sequence from reads that belong to each OTU and identify a family name and a name of genes/species. This allows the presence/absence and hit rate of bacteria that belong to a specific family to be measured.

The present invention may use labels or scores set on the basis of the presence/absence of bacteria that belong to Fusobacteriaceae and/or Veillonellaceae as information on the presence/absence of bacteria that belong to Fusobacteriaceae and/or Veillonellaceae.

Labels set on the basis of the presence/absence of bacteria are those set as appropriate in accordance with the presence/absence of bacteria. For example, a label "1" may be assigned when bacteria are present, and a label "0" may be assigned when bacteria are absent.

Scores set on the basis of the presence/absence of bacteria are those set as appropriate in accordance with the presence/absence of bacteria. For example, for a specific family, a score "+1" is given when bacteria that belong to the family are present, and a score "-1" is given when those bacteria are absent.

These scores may be calculated for each bacteria family and input to a reception means. Alternatively, it is possible to input the presence/absence of bacteria that belong to any bacteria family to a reception means and calculate scores for each bacteria family in accordance with a predetermined score assignment standard on the basis of the input data on the presence/absence of the bacteria.

A system for predicting disease contraction according to the invention also may be arranged so that an assessment means sums up the scores calculated or input for each bacteria family and predict and determine the possibility of contraction of a specific disease on the basis of the total score obtained.

### [Assessment means]

An assessment means of the present invention is a means for performing a predictive assessment regarding whether an animal will contract a specific disease within a predetermined period from information input to a reception means on the presence/absence of bacteria that belong to a predetermined bacteria family in a sample collected from the animal. A predictive assessment method is not particularly limited. For example, a processor performs a predictive assessment regarding whether an animal will contract a specific disease within a predetermined period from information on the presence/absence of bacteria that belong to a predetermined bacteria family in a sample collected from the animal. Or, as mentioned above, a configuration may be used where a score is calculated in accordance with a prespecified standard from the presence/absence of bacteria of each bacteria family and a risk of disease contraction is determined on the basis of the total score of sum of the above score. When the total score is equal to or more than a predetermined value, there is a high risk of disease contraction and a risk of disease contraction is low when the total score is less than the predetermined value.

An assessment means according to the invention, receiving information on the presence/absence of bacteria that belong to a predetermined bacteria family in a sample collected from an animal, performs a predictive assessment regarding whether the animal will contract a specific disease, preferably within a predetermined period, or more preferably within a predetermined period from the time of reception, from the time of collection of the sample, or from the time of reception of data on the presence/absence of bacteria that belong to a predetermined bacteria family. The predetermined period is preferably within three years, more preferably within two years, still more preferably within one year, and far more preferably within 180 days.

The assessment means according to the invention may be configured to perform a predictive assessment using a learned model. Such a learned model is preferably one that has leaned the relation between data on the presence/absence of bacteria that belong to Fusobacteriaceae and/or Veillonellaceae in a sample collected from an animal, and information on whether the animal has contracted a specific disease within a predetermined period from the time of acquisition of the sample or from the time of acquisition of data on the presence/absence of bacteria that belong to a predetermined bacteria family. The learned model is more preferably one that has used, as training data, in the learning process, data on the presence/absence of bacteria that belong to a predetermined bacteria family in a specimen collected from an animal, for example an intestinal microflora, and information on whether the animal has contracted a specific disease within a predetermined period from the time of acquisition of the sample or acquisition of data on the presence/absence of bacteria that belong to a predetermined bacteria family. The predetermined period required by the information, used for training data, on whether the animal has contracted a specific disease within a predetermined period, is preferably within three years, more preferably within two years, still more preferably within one year, and far more preferably within 180 days.

Artificial Intelligence (AI) is preferable as the learned model. Artificial Intelligence (AI) is software or a system that causes a computer to mimic intellectual work performed by human brains, and specifically refers to a computer program or the like that recognizes a natural language used by a human or performs logical inference or learns from experience. Artificial Intelligence may be either a general-purpose type or a specialized type, and may be any one of a deep neural network, a convolutional neural network, and the like, and disclosed software can be used.

In order to generate a learned model, artificial intelligence is caused to perform learning using training data. Learning may be either machine learning or deep learning, but deep learning is preferable. Deep learning is an advanced extension of machine learning, and is characterized in that a feature value is automatically found. The invention uses, as a feature value, data on the presence/absence of bacteria that belong to the family Fusobacteriaceae and/or Veillonellaceae in a sample collected from an animal, for example intestinal microflora.

A learning method for generating a learned model is not particularly limited, and disclosed software can be used. For example, DIGITS (the Deep Learning GPU Training System) disclosed by NVIDIA can be used. For example, learning may be taught by the well-known Support Vector Machine Method disclosed in "An introduction to support vector machine" (Kyoritsu Shuppan Co.) or the like.

Machine learning may be any of unsupervised learning and supervised learning, but supervised learning is preferable. Methods for supervised learning are not particularly limited and may be, for example, decision tree, ensemble learning, gradient boosting, and the like. Published algorithms for machine learning are, for example, XGBoost, CatBoost, and LightGBM.

The training data for learning are, for example, data on the presence/absence of bacteria that belong to Fusobacteriaceae and/or Veillonellaceae in a sample collected from an animal, for example intestinal microflora, and the presence/absence of disease contraction, that is, whether or not the animal has contracted a specific disease within a predetermined period, preferably within three years, more preferably within two years, still more preferably within one year, and far more preferably within 180 days, from the time of collection of the sample or from the time of acquisition of data on the presence/absence of bacteria that belong to a predetermined bacteria family. Whether the animal has contracted a specific disease may be replaced with a dummy variable. Data on the presence/absence of bacteria that belong to a predetermined bacteria family in a sample collected from an animal, as training data, are the same as data on the presence/absence of bacteria that belong to a predetermined bacteria family in an intestinal microflora as described in the above-mentioned reception method. Information on whether the animal has contracted a specific disease within a predetermined period is available, for example, from an animal hospital or a keeper that has signed up for insurance or the like, in relation to the fact of an insurance claim (also referred to as an "accident").

### [Output]

An output form of the predictive assessment by the assessment means according to the invention is not particularly limited, and the predictive assessment can be output by displaying, for example on the screen of a terminal such as a personal computer or smartphone, a message such as "Possibility of contracting keratoconjunctivitis sicca within one year", "High possibility of contracting allergic dermatitis within one year,," or "0% possibility of contracting inflammatory bowel disease within one year".

The system for predicting disease contraction may separately include an output means for receiving an assessment result from the assessment means and outputting the assessment result.

The system for predicting disease contraction may further include a proposal means for proposing a life improvement method in accordance with a result of prediction of disease contraction. For example, the proposal means may receive a result of prediction output from the assessment means, and in accordance with the result of the prediction, propose or recommend meals for avoiding a predicted risk of disease contraction, supplements containing disease prevention bacteria, low-salt, low-calorie meals, low carbohydrate meals, or diet meals. The proposal means may include a learned model.

In accordance with the result of the prediction output by the system for predicting disease contraction or the method for predicting disease contraction according to the invention, it is possible to manufacture or customize beverages, meals or supplements to prevent the contraction of a specific disease. As services related to the prediction of disease contraction, it is possible to perform prediction using the system for predicting disease contraction or the method for predicting disease contraction according to the invention, provide a result of prediction, manufacture or customize beverages meals, or supplements in accordance with a result of prediction, or propose the beverages, meals or supplements. After providing such services, it is also possible to implement the method for predicting disease contraction according to the invention and present whether the possibility of contracting a specific disease has decreased. The above beverages, meals and supplements include dietetic beverages, diet meals and dietary supplement additives.

In this way, there is hope for a reduction in or avoidance of the risk of contracting a specific disease through the proposal, manufacture, and customizing of meals or foods in accordance with the results of the prediction.

### [Family Fusobacteriaceae]

Fusobacteriaceae is a family that belongs to the order Fusobacteriales and includes the genus Fusobacterium.

### [Family Veillonellaceae]

Veillonellaceae is a family that belongs to the order Clostridiales and includes the genera such as Veillonellaceae, Acidaminococcus, Anaeroarcus, Anaerovibrio, Dialister, Megamonas, Megasphaera, Mitsuokella, Pectinatus, Phascolarctobacterium, Propionispira, and SelenomonasSucciniclasticum.

### [Other bacteria families]

The system for predicting disease contraction according to the invention may use, for predictive assessment, data on the presence/absence or hit rate of bacteria that belong to other families other than Fusobacteriaceae and Veillonellaceae.

Such other bacteria families include, but are not particularly limited, Campylobacteraceae, Clostridiaceae, Coprobacillaceae, Desulfovibrionaceae, Enterobacteriaceae, Enterococcaceae, Erysipelotrichaceae, Lachnospiraceae, Lactobacillaceae, Paraprevotellaceae, Porphyromonodaceae, Prevotellaceae, Turicibacteraceae, Comamonadaceae, Leuconostocaceae, Pseudomonadaceae, and Sphingobacteriaceae.

### [Family Campylobacteraceae]

Campylobacteraceae is a family that belongs to the class Epsilonproteobacteria, the order Campylobacterales, and includes the genus Campylobacter.

### [Family Clostridiaceae]

Clostridiaceae is a family that belongs to the order Clostridiales and includes the genus Clostridium.

### [Family Coprobacillaceae]

Coprobacillaceae is a family that belongs to the order Erysipelotrichales and includes the genus Coprobacillus.

### [Family Desulfovibrionaceae]

Desulfovibrionaceae is a family that belongs to the class Deltaproteobacteria, the order Desulfovibrionaceae, and includes the genus Desulfovibrionaceae.

### [Family Enterobacteriaceae]

Enterobacteriaceae is a family that belongs to the phylum Proteobacteria, the class Gammaproteobacteria, the order Enterobacterales. Enterobacteriaceae includes the genera such as Enterobacteriaceae, Escherichia, Klebsiella, Salmonella, Serratia, Yersinia, the genus Arsenophonus, Biostraticola, Candidatus Blochmannia, Brenneria, Buchnera, Budvicia, Buttiauxella, Cedecea, Citrobacter, Cosenzaea, Cronobacter, Dickeya, Edwardsiella, and Erwinia.

### [Family Enterococcaceae]

Enterococcaceae is a family of gram-positive eubacteria that belongs to the order Lactobacillales. Representative genera include Enterococcus, Melissococcus, Pilibacter, Tetragenococcus, and Vagococcus.

### [Family Erysipelotrichaceae]

Erysipelotrichaceae is a family that belongs to the order Erysipelotrichales and includes the genera such as Allobaculum, Bulleidia, Erysipelothrix, and Holdemania.

### [Family Lachnospiraceae]

Lachnospiraceae is a family that belongs to the class Clostridia, the order Clostridiales, and includes the genus Lachnospira.

### [Family Lactobacillaceae]

Lactobacillaceae is a family that belongs to the order Lactobacillales and includes the genus Lactobacillus.

### [Family Paraprevotellaceae]

Paraprevotellaceae is a family that belongs to the order Bacteroidales and includes the genus Paraprevotella. According to an alternative classification method, Paraprevotellaceae is not an independent family and the genus Paraprevotella is included in the family Prevotellaceae.

### [Family Porphyromonodaceae]

Porphyromonodaceae is a family that belongs to the order Bacteroidales and includes the genus Porphyromonas.

### [Family Prevotellaceae]

Prevotellaceae is a family that belongs to the order Bacteroidales and includes the genus Prevotella.

### [Family Turicibacteraceae]

Turicibacteraceae is a family that belongs to the order Turicibacterales and includes the genus Turicibacter.

### [Family Comamonadaceae]

Comamonadaceae is a family that belongs to the order Burkholderiales and includes the genus Comamonas. The genus Comamonas is a new genus including bacteria belonging to the genus Pseudomonas that have been re-classified via systematic analysis of genes.

### [Family Leuconostocaceae]

Leuconostocaceae is a gram-positive bacteria family that belongs to the order Lactobacillales. Representative genera include Fructobacillus, Leuconostoc, Oenococcus, and Weissella.

According to an alternative classification method, the family Leuconostocaceae is not an independent family and the genus Leuconostoc is included in the family Lactobacillaceae.

### [Family Pseudomonadaceae]

Pseudomonadaceae is a family that belongs to the order Pseudomonadales and includes the genus Pseudomonas.

### [Family Sphingobacteriaceae]

Sphingobacteriaceae is a family that belongs to the order Sphingobacteriales and includes the genus Sphingobacterium.

The system for predicting disease contraction according to the invention may use a predetermined bacteria family may use, on top of data on the presence/absence or hit rate of bacteria that belong to a specific bacteria family, information such as a facial image, kind, breed, age, gender, weight, past medical history, gene sequence information, SNP, and the presence/absence of genetic mutation of an animal.

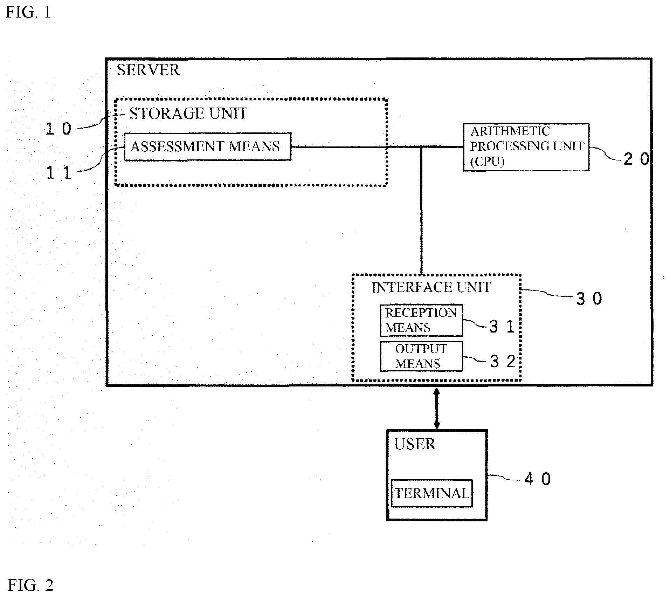

An embodiment of the system for predicting disease contraction according to the invention will be described with reference to FIG. 1.

In FIG. 1, a terminal 40 is one used by a person (user) that wishes to use the system for predicting disease contraction. The terminal 40 may be a personal computer, a smartphone, a tablet terminal, or the like. The terminal 40 includes a processing unit such as a CPU, a hard disk, a storage unit such as a ROM or a RAM, a display unit such as a liquid crystal panel, an input unit such as a mouse, a keyboard and a touch panel, and a communication unit such as a network adapter.

The user accesses a server from the terminal 40 and then inputs and sends information on the presence/absence of bacteria that belong to the family Fusobacteriaceae and/or Veillonellaceae in a sample collected from an animal, and as required, information including a facial image (photograph), kind, breed, age, weight and past medical history of the animal.

The user is able to receive a result of the prediction of disease contraction in the server upon access of the terminal 40 to the server.

The user receives a feces sample collection kit sent for investigating a bacterial flora in a sample, for example, feces, collected from an animal caretaken by the user, and sends the feces sample to a business operator that performs measurement of a bacterial flora (not illustrated). The business operator measures the bacterial flora in the feces sample of the animal and acquires data on the presence/absence of bacteria that belong to Fusobacteriaceae and/or Veillonellaceae in the sample. The business operator may directly input and send, via its own terminal, to a reception means 31 of the server, data on the presence/absence of bacteria that belong to Fusobacteriaceae and/or Veillonellaceae in the feces sample of the animal. The business operator may separately send to the user, via mail, e-mail, or the like, data on the presence/absence of bacteria that belong to Fusobacteriaceae and/or Veillonellaceae in the feces sample of the animal, and the user may input and send the data to the reception means 31 via the terminal 40. When the business operator receives the feces, analyzes bacterial flora and inputs an analysis result to the system for predicting disease contraction according to the invention, the user needs only to collect and send feces of an animal in his/her care via mail to know the possibility that the animal will contract a specific disease.

While the server consists of a computer in this embodiment, the server may be any apparatus as long as the apparatus has functions according to the invention.

The storage unit 10 includes, for example, a ROM, a RAM, a hard disk or the like. An information processing program to operate each unit of the server is stored in the storage unit 10, and in particular, software for the assessment means 11, or the like, is stored therein.

As mentioned above, the assessment means 11 uses, as an input, data input by the user or the business operator that has measured the bacterial flora of the target animal on the presence/absence of bacteria that belong to Fusobacteriaceae and/or Veillonellaceae, and outputs a prediction that the animal will contract a specific disease within a predetermined period (for example within one year) or the percentage of the possibility of contraction. The assessment means may be a learned model. Such a learned model includes, for example, XGBoost, CatBoost, LightGBM, or a deep neural network or a convolutional neural network.

While an assessment means and a reception means are stored in a server and connected to a user's terminal via a connection means such as the Internet or LAN in this embodiment, the invention is not limited thereto, but a configuration is possible where an assessment means, a reception means and an interface unit are stored in a single server or apparatus, or a configuration may be employed that does not separately require a terminal used by the user.

### <Method for predicting disease contraction>

A method for predicting disease contraction according to the invention is characterized by including a step of performing predictive assessment regarding the possibility of contracting keratoconjunctivitis sicca, allergic dermatitis or inflammatory bowel disease of an animal from information on the presence/absence of bacteria that belong to Fusobacteriaceae and/or Veillonellaceae in a sample collected from the animal. Preferably, the method includes a step of preparing information on the presence/absence of bacteria that belong to Fusobacteriaceae and/or Veillonellaceae in a sample collected from an animal, and a step of performing predictive assessment regarding the possibility of contracting keratoconjunctivitis sicca, allergic dermatitis or inflammatory bowel disease. The method may be carried out, for example, using the above-mentioned system for predicting disease contraction.

The information on the presence/absence of bacteria that belong to Fusobacteriaceae and/or Veillonellaceae in a sample collected from an animal, as used by the method for predicting disease contraction according to the invention, and a method for performing predictive assessment regarding the possibility of contracting a specific disease within a predetermined period from the information on the presence/absence of bacteria that belong to Fusobacteriaceae and/or Veillonellaceae in a sample collected from the animal, and a configuration therefor, are the same as those described in relation to the above-mentioned system for predicting disease contraction.

FIG. 2 illustrates a flowchart of predictive assessment of disease contraction based on an embodiment of a method for predicting disease contraction using a system for predicting disease contraction according to the invention. The embodiment is described, for the purpose of ease of explanation, including acquisition of a sample from an animal and acquisition of data on a bacterial flora in the sample, for example an intestinal microflora. The user collects a feces sample from an animal using a feces collection kit or the like and sends the sample to an intestinal microflora analysis business operator (step S1). The intestinal microflora analysis business operator uses a next-generation sequencer to analyze and acquire data on the presence/absence of bacterial that belong to a predetermined bacteria family in the intestinal microflora of the animal from the feces sample (step S2). The intestinal microflora analysis business operator sends back the data on the intestinal microflora to the user. The user accesses the system for predicting disease contraction via a terminal and inputs data on the presence/absence of bacteria that belong to Fusobacteriaceae and/or Veillonellaceae in the intestinal microflora of the animal (step S3). The system for predicting disease contraction performs predictive assessment regarding the possibility that the animal will contract a specific disease within a predetermined period (for example within one year) from the input data on the presence/absence of bacteria that belong to Fusobacteriaceae and/or Veillonellaceae in the intestinal microflora of the animal (step S4). The system for predicting disease contraction outputs the predictive assessment and sends the predictive assessment to the terminal 40, on which a result of the predictive assessment is displayed (step S5).

### <Insurance fee calculation system>

An insurance fee calculation system according to the invention includes: a reception means for receiving information on the presence/absence of bacteria that belong to Fusobacteriaceae and/or Veillonellaceae in a sample collected from an animal; and an insurance fee calculation means for calculating or correcting an insurance fee using the information, input to the reception means, on the presence/absence of bacteria that belong to Fusobacteriaceae and/or Veillonellaceae.

The inventors have analyzed huge data on the bacterial flora in samples collected from animals insured by pet insurance, in particular bacterial flora in feces samples and the presence/absence of insurance claims insured animals, and as a result, they found that there is a correlation between the presence/absence of bacteria that belong to Fusobacteriaceae and/or Veillonellaceae and the insurance claim amount and insurance claim count. Accordingly, using whether an animal going to be insured by pet insurance carries bacteria that belongs to Fusobacteriaceae and/or Veillonellaceae as a ground for insurance fee calculation, it is possible to calculate an insurance fee reflecting the possibility of a future insurance claim and correct the calculated insurance fee using other elements such as a breed, age, gender, weight, past medical history, and the like.

### [Reception means]

A reception means in the insurance fee calculation system is the same as the above-mentioned reception means in the system for predicting disease contraction.

### [Insurance fee calculation means]

An insurance fee calculation means is a means for calculating a proper insurance fee for an animal from information, input to a reception means, on the presence/absence of bacteria that belong to a predetermined bacteria family in a sample collected from the animal, and as required, information such as a breed, age, gender, weight and past medical history of the animal. An insurance fee calculation means is not particularly limited. For example, a processor uses preconfigured software or programs to calculate a proper insurance fee for an animal from information on the presence/absence of bacteria that belong to a predetermined bacteria family in a sample collected from the animal. In calculating an insurance fee, a prestored insurance fee table may be referenced. For example, software constituting an insurance fee calculation means may be software for classifying the insurance fee into a grade in accordance with a kind, breed, gender, weight, past medical history and the like of the animal, and finally correcting the grade by taking into account the presence/absence of bacteria that belong to Fusobacteriaceae and/or Veillonellaceae and calculating a final insurance fee.

### <Insurance fee calculation method>

An insurance fee calculation method according to the invention is characterized by including a step of calculating or correcting an insurance fee using information on the presence/absence of bacteria that belong to Fusobacteriaceae and/or Veillonellaceae in a sample collected from an animal. Preferably, the method includes a step of preparing information on the presence/absence of bacteria that belong to Fusobacteriaceae and/or Veillonellaceae in a sample collected from an animal, and a step of calculating or correcting an insurance fee on the basis of the information. The method may be carried out, for example, using the above-mentioned insurance fee calculation system.

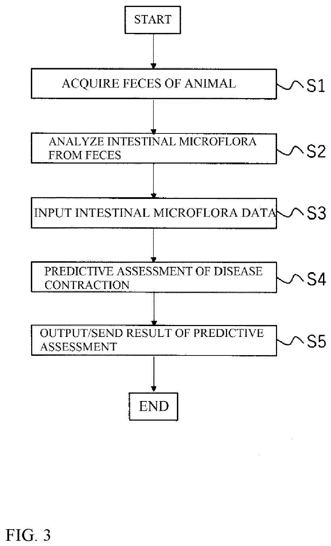

An embodiment of an insurance fee calculation system according to the invention will be described with reference to FIG. 3. Description of parts in common with FIG. 1 is omitted as appropriate.

The user receives a feces sample collection kit sent for testing a bacterial flora in a sample, for example, feces, collected from an animal caretaken by the user and sends the feces sample collected back to a business operator that performs measurement of a bacterial flora (not illustrated). The business operator measures the bacterial flora in the feces sample of the animal and acquires data on the presence/absence of bacteria that belong to Fusobacteriaceae and/or Veillonellaceae in the sample. The business operator may directly input and send, via its own terminal, to a reception means 31 of the server, data on the presence/absence of bacteria that belong to Fusobacteriaceae and/or Veillonellaceae in the feces sample of the animal. The business operator may separately send to the user, via mail, e-mail, or the like, data on the presence/absence of bacteria that belong to Fusobacteriaceae and/or Veillonellaceae in a feces sample of the animal, and the user may input and send the data to the reception means 31 via the terminal 40. When the business operator receives the feces, analyzes bacterial flora and inputs an analysis result to the system for predicting disease contraction according to the invention, the user needs only to collect and send feces of an animal via mail to know the possibility that the animal will contract a specific disease.

In FIG. 3, an insurance fee calculation means 12 is software for calculating an insurance fee of an animal from information input by the user such as a kind, breed, age at the time of acquisition of bacterial flora data, weight and past medical history of the animal. For example, the software is software for classifying an insurance fee into a grade in accordance with a kind, breed, age at the time of acquisition of intestinal microflora data, weight, past medical history and the like of the animal, and finally correcting the grade by taking into account the presence/absence of bacteria that belong to Fusobacteriaceae and/or Veillonellaceae and calculating a final insurance fee on the basis of an insurance fee table 13.

Separately from an insurance fee calculation means 12, an assessment means 11 for predicting contraction of a specific disease may be provided. An insurance fee calculation means 12 and an assessment means 11 may be all together a piece of software.

An arithmetic processing unit 20 predicts contraction of a disease and calculates an insurance fee using the assessment means 11 and the insurance fee calculation means 12 that are stored in the storage unit.

An interface unit 30 includes a reception means 31 and an output means 32, receives data on bacteria that belong to a predetermined bacteria family in a bacterial flora, for example, an intestinal microflora in a sample of an animal as well as other information from the user's terminal, and outputs prediction of disease contraction and a result of calculation of an insurance fee to the user's terminal.

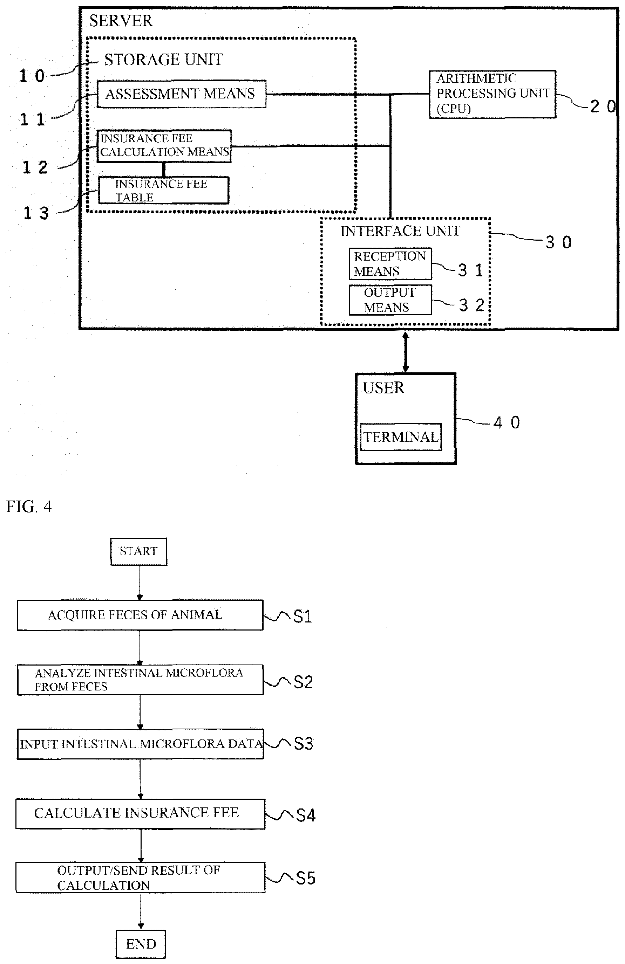

FIG. 4 illustrates a flowchart of predictive assessment of disease contraction based on an embodiment of an insurance fee calculation method using an insurance fee calculation system according to the invention. The embodiment is described, for the purpose of ease of explanation, including acquisition of a sample from an animal and acquisition of data on a bacterial flora in the sample, for example an intestinal microflora. The user collects a feces sample from an animal using a feces collection kit or the like and sends the sample to an intestinal microflora analysis business operator (step S 1). The intestinal microflora analysis business operator uses a next-generation sequencer to analyze and acquire data on the presence/absence of bacterial that belong to a predetermined bacteria family in the intestinal microflora of the animal from the feces sample (step S2). The intestinal microflora analysis business operator sends back the data on the intestinal microflora to the user. The user accesses the system for predicting disease contraction via a terminal and inputs data on the presence/absence of bacteria that belong to Fusobacteriaceae and/or Veillonellaceae in the intestinal microflora of the animal (step S3). The insurance fee calculation system calculates an insurance fee of the animal from the input data on the presence/absence of bacteria that belong to Fusobacteriaceae and/or Veillonellaceae in the intestinal microflora of the animal (step S4). The insurance fee calculation system outputs the predictive assessment and sends the predictive assessment to the terminal 40, on which a calculation result is displayed (step S5).

### EXAMPLES

Examples according to the invention are described below. Note that the invention is not limited to the following examples.

### (Extraction of DNA from feces sample)

A faces sample was collected from each dog and DNA was extracted therefrom as follows:
A dog keeper collected a feces sample of the dog using a feces collection kit. The feces sample was received and suspended in water.

Next, a feces-suspended liquid of 200 µL and a lysis buffer of 810 µL (containing Protenase K of 224 µg/mL) were added to a bead tube and bead crushing (6,000 rpm, crushing 20 seconds, interval 30 seconds, crushing 20 seconds) was performed using a bead homogenizer. After that, a specimen was left still for 10 minutes on a heat block at a temperature of 70°C to perform treatment via Protenase K, and then left still for 5 minutes on the heat block at a temperature of 95°C to inactivate Protenase K. From the specimen, after undergoing lysis treatment, DNAs were automatically extracted via the protocol for chemagic stool kit using chemagic360 (PerkinElmer) to obtain a DNA extract liquid of 100 µL.

### (Meta-16 sRNA gene sequence analysis)

Meta-16 sRNA gene sequence analysis was carried out by modifying Illumina 16s Metagenomic Sequencing Library Preparation (Version 15044223-B). First, a region 460bp including variable region V3-V4 of 16 SrNA genes was amplified with PCR using a universal primer (Illumina_16S_341F and Illumina_16S_805RPCR). A PCR reaction liquid was prepared by mixing a DNA extract liquid of 10 µL, each primer of 0.05 µL (100 µM), 2xKAPA HiFi Hot-Start Ready Mix (F.Hoffmann-LaRoche, Switzerland) of 12.5 µL, and PCR grade water of 2.4 µL. During PCR process, heat denaturation took place at 95°C for three minutes and then a cycle of 95°C for 30 seconds, 55°C for 30 seconds, and 72°C for 30 seconds was repeated 30 times, and finally elongation reaction took place at 72°C for five seconds. An amplification product was refined using magnetic beads and was eluted with Buffer EB (QIAGEN, Germany) of 50 µL. Post-refinement amplification product underwent PCR using Nextera XT Index Kit v2 (illumine, CA, US) and an index was added. A PCR reaction liquid was prepared by mixing an amplification product of 2.5 µL, each primer of 2.5µL, 2x KAPA HiFi Hot-Start ReadyMix of 12.5 µL and PCR grade water of 5 µL. During PCR process, heat denaturation took place at 95°C for three minutes and then a cycle of 95°C for 30 seconds, 55°C for 30 seconds, and 72°C for 30 seconds was repeated 12 times, and finally elongation reaction took place at 72°C for five seconds. An indexed amplification product was refined using magnetic beads and was eluted with Buffer EB of 80-105 µL. Concentration of each amplification product was measured using Nano Photometer (Implen, CA, US), prepared at 1.4 nM, and the products were mixed together in equal amounts to prepare a library for sequence. DNA concentration of the library for sequence and size of amplification products were confirmed via electrophoresis, and were analyzed with MiSeq. Analysis used MiSeq Reagent Kit V3 with pair end sequencing of 2×300 bp taking place. The obtained sequence was analyzed with MiSeq Reporter to obtain bacterial composition data.

The sequence of the universal primer used above is as shown below. The universal primer is available from the market.
Illumina_16S_341F
   5'-TCGTCGGCAGCGTCAGATGTGTATAAGAGACAGCCTACGGGNGGCWGCAG- 3'
llumina_16S_805R

5′-
GTCTCGTGGGCTCGGAGATGTGTATAAGAGACAGGACTACHVGGGTATCTAATCC
- 3’

In accordance with the above-mentioned method, intestinal microflora composition data was obtained concerning 19040 Toy Poodle individuals over the age of 0 (average age: 3.4 years) to measure the presence/absence of bacteria that belong to a predetermined bacteria family in the intestinal microflora. Among the 19040 individuals, the number of individuals that made an insurance claim (accident group) within 180 days before and after collection of feces samples (total 360 days) was 7805. The number of individuals that did not make an insurance claim (no-accident group) was 11235.

### [Example 1]

Among the above-mentioned 19040 individuals, the presence/absence of bacteria that belong to Fusobacteriaceae and Veillonellaceae was investigated regarding toy poodles of the accident group, and the relation between the presence/absence of those bacteria and the annual insurance payment amount was investigated.

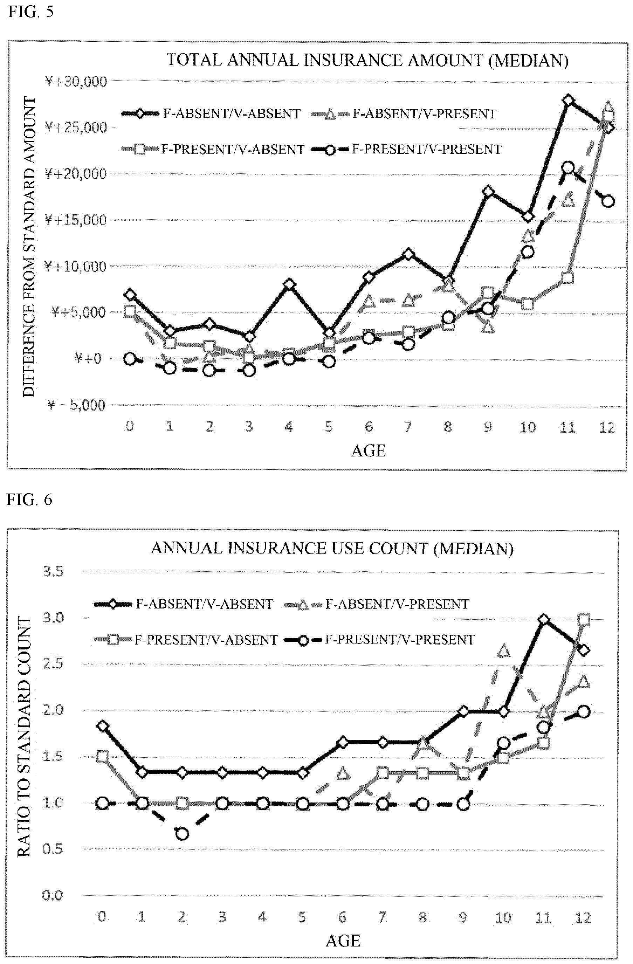

The result is illustrated in FIG. 5.

In FIG. 5, "F-Absent/V-Absent" refers to a population that carries neither bacteria that belong to Fusobacteriaceae nor bacteria that belong to Veillonellaceae.

"F-Absent/V-Present" refers to a population that does not carry bacteria that belong to Fusobacteriaceae but carries bacteria that belong to Veillonellaceae.

"F-Present/V-Absent" refers to a population that carries bacteria that belong to Fusobacteriaceae but does not carry bacteria that belong to Veillonellaceae.

"F-Present/V-Present" refers to a population that carries both bacteria that belong to Fusobacteriaceae and bacteria that belong to Veillonellaceae.

As clear from FIG. 5, it is understood that, in all age groups, the population of F-Absent/V-Absent has a higher total annual insurance amount and thus a larger amount of insurance payout. On the other hand, the population of F-Present/V-Present tends to have a lower total annual insurance amount and a smaller amount of insurance payout (the amount for the population of F-Present/V-Present at the age of 0 was used as the standard amount).

In this way, it is confirmed that there is a certain correlation between the presence/absence of bacteria that belong to Fusobacteriaceae and Veillonellaceae in the feces sample of the animal and the amount of insurance claims paid.

### [Example 2]

Among the above-mentioned 19040 individuals, the presence/absence of bacteria that belong to Fusobacteriaceae and Veillonellaceae was investigated regarding toy poodles of the accident group, and a relation between the presence/absence of those bacteria and an annual insurance use count was investigated.

The result is illustrated in FIG. 6.

As clear from FIG. 6, it is understood that, in all age groups, the population of F-Absent/V-Absent has a higher number of annual insurance use, that is, pet insurance is more often used for the treatment of an animal in an animal hospital. On the other hand, the population of F-Present/V-Present tends to have a lower number of annual insurance use and thus tends to use pet insurance less often (the number of the population of F-Present/V-Present at the age of 0 was used as the standard value 1).

In this way, it is confirmed that there is a certain correlation between the presence/absence of bacteria that belong to Fusobacteriaceae and Veillonellaceae in the feces sample of the animal, and the number of insurance uses.

### [Example 3]

Regarding the above-mentioned 19040 individuals, the presence/absence of bacteria that belong to Fusobacteriaceae and Veillonellaceae was investigated, and a relation between the presence/absence of those bacteria and the presence/absence of contraction of keratoconjunctivitis sicca was investigated. Note that, among a total of 19040 individuals, 22 individuals were confirmed to have contracted keratoconjunctivitis sicca within 180 days before and after collection of feces samples (total 360 days).

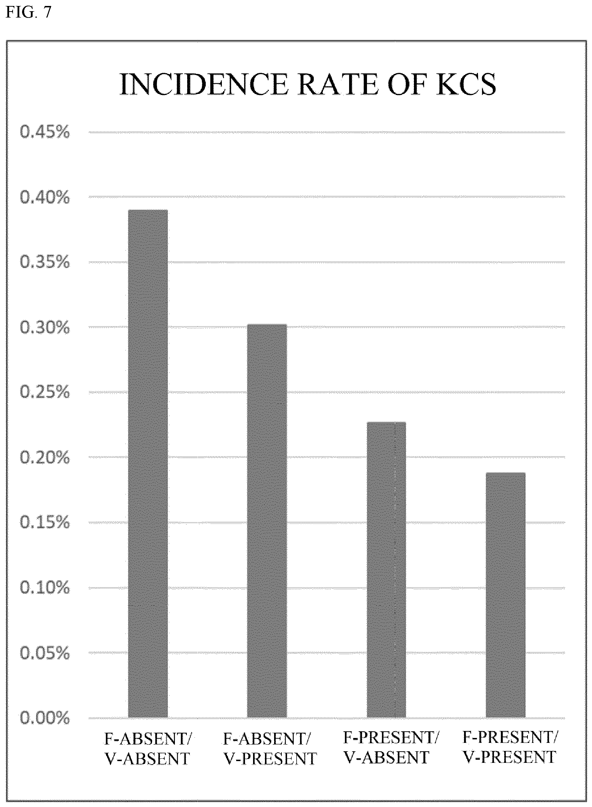

Regarding each of the populations of F-Absent/V-Absent, F-Absent/V-Present, F-Present/V-Absent, and F-Present/V-Present, an incidence rate of keratoconjunctivitis sicca (the rate of individuals that have contracted keratoconjunctivitis sicca in each population) was calculated. The result is illustrated in FIG. 7.

As clear from FIG. 7, the incidence rate of keratoconjunctivitis sicca dropped in a descending order of F-Absent/V-Absent, F-Absent/V-Present, F-Present/V-Absent, and F-Present/V-Present. There is thus found a possibility of describing a relation between the presence/absence of bacteria that belong to Fusobacteriaceae and/or Veillonellaceae, and keratoconjunctivitis sicca. Moreover, it is found possible to perform predictive assessment of whether an animal will contract keratoconjunctivitis sicca by investigating the presence/absence of bacteria that belong to Fusobacteriaceae and/or Veillonellaceae.

### [Example 4]

Regarding the above-mentioned 19040 individuals, the presence/absence of bacteria that belong to Fusobacteriaceae and Veillonellaceae were investigated and a relation between the presence/absence of those bacteria and the presence/absence of contraction of allergic dermatitis was investigated. Note that, among a total of 19040 individuals, 444 individuals were confirmed to have contracted allergic dermatitis within 180 days before and after collection of feces samples (total 360 days).

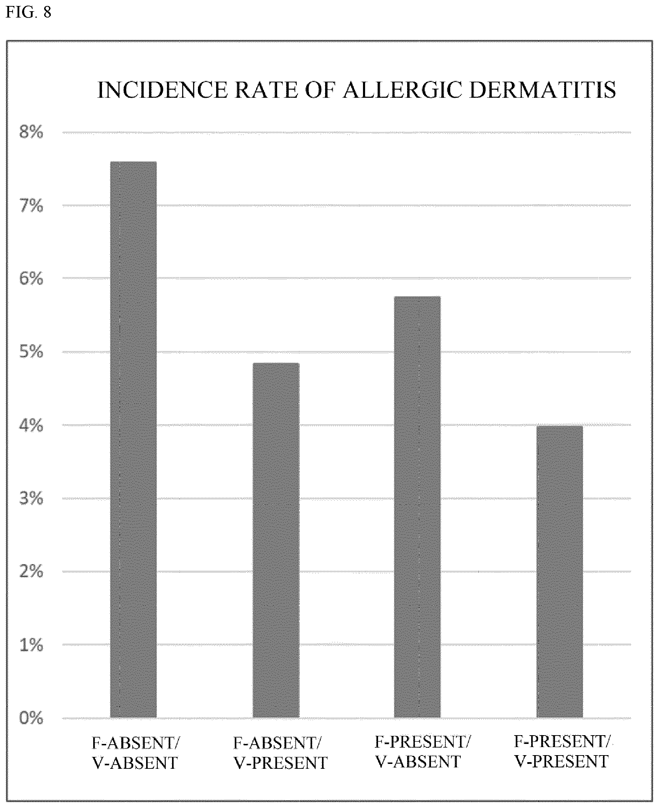

Regarding each of the populations of F-Absent/V-Absent, F-Absent/V-Present, F-Present/V-Absent, and F-Present/V-Present, an incidence ratio of allergic dermatitis (the rate of individuals that have contracted allergic dermatitis in each population) was calculated. The result is illustrated in FIG. 8.

As clear from FIG. 8, the incidence rate of allergic dermatitis dropped in a descending order of F-Absent/V-Absent, F-Present/V-Absent, F-Absent/V-Present, and F-Present/V-Present. There is thus found a possibility of indicating a relation between the presence/absence of bacteria that belong to Fusobacteriaceae and/or Veillonellaceae and allergic dermatitis. Moreover, it is found possible to perform a predictive assessment of whether an animal will contract allergic dermatitis by investigating the presence/absence of bacteria that belong to Fusobacteriaceae and/or Veillonellaceae.

### [Example 5]

Regarding the above-mentioned 19040 individuals, the presence/absence of bacteria that belong to Fusobacteriaceae and Veillonellaceae was investigated, and a relation between the presence/absence of those bacteria and the presence/absence of contraction of inflammatory bowel disease was investigated. Note that, among a total of 19040 individuals, 12 individuals were confirmed to have contracted inflammatory bowel disease within 180 days before and after collection of feces samples (total 360 days).

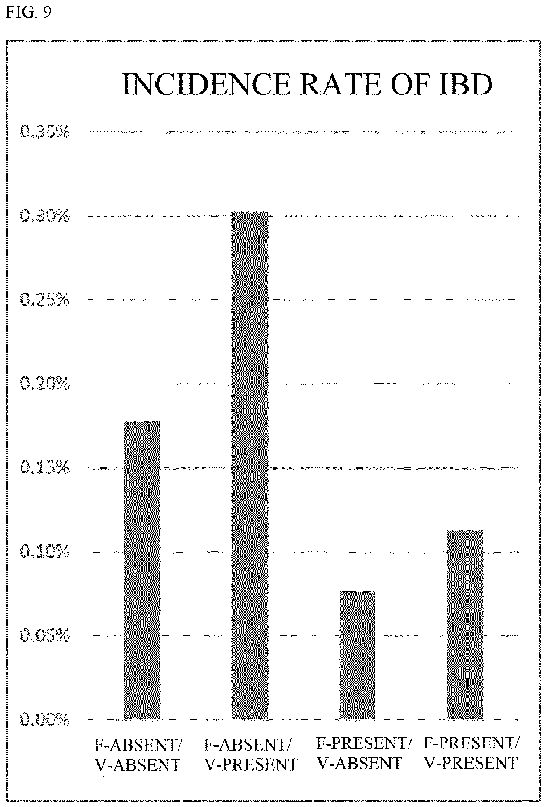

Regarding each of the populations of F-Absent/V-Absent, F-Absent/V-Present, F-Present/V-Absent, and F-Present/V-Present, an incidence rate of inflammatory bowel disease (the rate of individuals that have contracted inflammatory bowel disease in each population) was calculated. The result is illustrated in FIG. 9.

As clear from FIG. 9, the incidence rate of inflammatory bowel disease dropped in a descending order of F-Absent/V-Present, F-Absent/V-Absent, F-Present/V-Present, and F-Present/V-Absent. There is thus found a possibility of indicating a relation between the presence/absence of bacteria that belong to Fusobacteriaceae and/or Veillonellaceae and inflammatory bowel disease. Moreover, it is found possible to perform a predictive assessment of whether an animal will contract inflammatory bowel disease by examining the presence/absence of bacteria that belong to Fusobacteriaceae and/or Veillonellaceae.

## Claims

1. A system for predicting disease contraction comprising: a reception means for receiving information on the presence/absence of bacteria belonging to Fusobacteriaceae and/or Veillonellaceae in a sample collected from an animal; and an assessment means for performing predictive assessment regarding the possibility of the animal contracting keratoconjunctivitis sicca from the information, input to the reception means, on the presence/absence of bacteria belonging to Fusobacteriaceae and/or Veillonellaceae.

2. A system for predicting disease contraction comprising: a reception means for receiving information on the presence/absence of bacteria belonging to Fusobacteriaceae and/or Veillonellaceae in a sample collected from an animal; and an assessment means for performing predictive assessment regarding the possibility of the animal contracting allergic dermatitis from the information, input to the reception means, on the presence/absence of bacteria belonging to Fusobacteriaceae and/or Veillonellaceae.

3. A system for predicting disease contraction comprising: a reception means for receiving information on the presence/absence of bacteria belonging to Fusobacteriaceae and/or Veillonellaceae in a sample collected from an animal; and an assessment means for performing predictive assessment regarding the possibility of the animal contracting inflammatory bowel disease from the information, input to the reception means, on the presence/absence of bacteria belonging to Fusobacteriaceae and/or Veillonellaceae.

4. The system for predicting disease contraction according to any one of claims 1 to 3, wherein the sample is feces.

5. An insurance fee calculation system comprising: a reception means for receiving information on the presence/absence of bacteria belonging to Fusobacteriaceae and/or Veillonellaceae in a sample collected from an animal; and an insurance fee calculation means for calculating or correcting an insurance fee using the information, input to the reception means, on the presence/absence of bacteria belonging to Fusobacteriaceae and/or Veillonellaceae.

6. A method for predicting disease contraction comprising performing predictive assessment regarding the possibility of an animal contracting keratoconjunctivitis sicca from information on the presence/absence of bacteria belonging to Fusobacteriaceae and/or Veillonellaceae in a sample collected from the animal.

7. A method for predicting disease contraction comprising performing predictive assessment regarding the possibility of an animal contracting allergic dermatitis from information on the presence/absence of bacteria belonging to Fusobacteriaceae and/or Veillonellaceae in a sample collected from the animal.

8. A method for predicting disease contraction comprising performing predictive assessment regarding the possibility of an animal contracting inflammatory bowel disease from information on the presence/absence of bacteria belonging to Fusobacteriaceae and/or Veillonellaceae in a sample collected from the animal.

9. The method for predicting disease contraction according to any one of claims 6 to 8, wherein the sample is feces.

10. An insurance fee calculation method comprising calculating or correcting an insurance fee using information on the presence/absence of bacteria belonging to Fusobacteriaceae and/or Veillonellaceae in a sample collected from an animal.
